(19) Europäisches Patentamt / European Patent Office / Office européen des brevets

(11) EP 2 095 287 B1

(12) FASCICULE DE BREVET EUROPEEN

(45) Date de publication et mention
de la délivrance du brevet:
**30.05.2018 Bulletin 2018/22**

(21) Numéro de dépôt: **07866550.2**

(22) Date de dépôt: **30.11.2007**

(51) Int Cl.:
***G06F 19/00*** *(2018.01)*

(86) Numéro de dépôt international:
**PCT/FR2007/052430**

(87) Numéro de publication internationale:
**WO 2008/065314 (05.06.2008 Gazette 2008/23)**

(54) **Procédé de vérification de l'état et des performances des appareils d'analyse biologique délocalisée**

Verfahren zur Überprüfung des Status und der Leistung von delokalisierten biologischen Analysegeräten

Method for verifying the status and performance of off-site biological analysis apparatuses

(84) Etats contractants désignés:
**AT BE BG CH CY CZ DE DK EE ES FI FR GB GR HU IE IS IT LI LT LU LV MC MT NL PL PT RO SE SI SK TR**

(30) Priorité: **01.12.2006 FR 0610529**

(43) Date de publication de la demande:
**02.09.2009 Bulletin 2009/36**

(73) Titulaire: **Accessible Conseils SARL
59310 Beuvry La Foret (FR)**

(72) Inventeurs:
• **DESJOBERT, Hélène
75015 Paris (FR)**
• **PODVIN, Jean
59310 Beuvry La Foret (FR)**

(74) Mandataire: **Domange, Maxime et al
Cabinet Beau de Lomenie
232, avenue du Prado
13295 Marseille Cedex 08 (FR)**

(56) Documents cités:
US-A- 5 835 384       US-A1- 2004 078 162
US-A1- 2004 220 761   US-A1- 2005 177 345
US-B1- 6 787 361

• PIAZZA L ET AL: "Avancees technologiques et integration de la biologie delocalisee dans les etablissements de soins", ITBM-RBM, EDITIONS SCIENTIFIQUES ET MEDICALES ELSEVIER, vol. 27, no. 2, 1 May 2006 (2006-05-01), pages 80-88, XP028073098, ISSN: 1297-9562 [retrieved on 2006-05-01]

EP 2 095 287 B1

**Description**

Arrière-plan de l'invention

La présente invention se rapporte au domaine général des appareils d'analyse biologique aptes à mesurer ou à évaluer une variable biologique de manière délocalisée.

De tels appareils sont généralement installés en dehors d'un laboratoire et bien souvent en service ambulatoire voire chez un patient. Ce sont généralement des appareils portables, parfois précalibrés, rapides et aisés d'utilisation.

L'invention s'applique plus particulièrement à tous les appareils d'analyse délocalisée de la glycémie mais concerne également l'analyse délocalisée d'autres paramètres de biologie dont la mesure serait susceptible d'être effectuée par un appareil d'analyse délocalisée et, donc, en dehors du laboratoire. Parmi de tels paramètres, on trouve les gaz du sang, la mesure de l'hématocrite, la mesure des lactates, la mesure de l'ionogramme sanguin ou urinaire, la mesure du glucose urinaire, la mesure de l'acide urique, la mesure du cholestérol, la mesure des triglycérides ainsi que tout autre paramètre physico-chimique et/ou biologique utile au diagnostic et à la prise de décision quant aux soins à prodiguer à un patient.

En fonction de leur construction, de tels appareils d'analyse délocalisée peuvent avoir un fonctionnement discret ou en continu.

L'invention s'intéresse particulièrement à de tels appareils d'analyse délocalisée pour lesquels un suivi de la qualité s'avère utile, voire nécessaire. En effet, ces appareils étant utilisés dans des lieux distincts, parfois privés, il est nécessaire de vérifier que les valeurs données par de tels appareils sont correctes.

Aujourd'hui, pour contrôler un tel appareil d'analyse délocalisée, une mesure dont le résultat est envoyé à un laboratoire est réalisée à l'aide de cet appareil et un prélèvement d'un sang veineux est réalisé simultanément pour être envoyé au laboratoire en même temps que la mesure réalisée par l'appareil d'analyse délocalisée.

Un biologiste fait alors une mesure de la valeur biologique dans le sang veineux puis compare les valeurs obtenues afin de déterminer si l'appareil d'analyse délocalisée présente un fonctionnement correct ou présente un fonctionnement erroné.

Dans la mesure où, généralement, dans le cas des appareils d'analyse délocalisée mesurant la glycémie, le sang analysé est généralement capillaire et, dans la mesure où le prélèvement envoyé au laboratoire est en revanche un sang veineux, il existe nécessairement un biais entre les valeurs mesurées pour ces deux types de sang.

Ce biais est rendu incertain et est majoré par le fait que les moyens d'analyse sont nécessairement distincts et font appel à des principes différents. L'incertitude sur la valeur réelle du biais entraine un risque si l'appareil d'analyse délocalisée est déclaré valide et qu'en réalité les mesures qu'il permet d'effectuer sont substantiellement fausses. En fait, le contrôle ainsi effectué ne peut donc être qu'approximatif et finalement très relatif à l'expertise du biologiste.

Il existe aujourd'hui un autre moyen pour contrôler un appareil d'analyse délocalisée préconisé par les fabricants de tels appareils. Ce contrôle est basé sur l'utilisation d'une solution proposée par le fabricant pour effectuer un contrôle d'analyse délocalisée.

Une fois une mesure acquise pour cette solution du fabricant, la valeur mesurée est comparée à des fourchettes fournies par ce fabricant.

Le contrôle est ici direct mais ne permet pas une évaluation de l'appareil de l'analyse délocalisée par rapport à d'autres appareils d'autres types et dont le fonctionnement peut être basé sur d'autres principes.

Il est également nécessaire d'être muni des solutions de contrôle particulières à chaque type d'appareil pour faire un contrôle sur un ensemble d'appareils d'analyse délocalisée. Cela est délicat à mettre en oeuvre et est source d'erreur. Cela peut donc être préjudiciable lorsque plusieurs types d'appareils d'analyse délocalisée sont utilisés dans un même service hospitalier et que leur fiabilité et leur homogénéité doivent être contrôlée.

L'invention se propose donc de fournir un procédé qui complète le contrôle de l'appareil d'analyse délocalisée avec la solution fournie par le fabricant et propose pour cela une solution technique automatisée améliorant et simplifiant le contrôle par mesures parallèles sur l'appareil d'analyse délocalisée et sur un appareil d'analyse situé dans un laboratoire.

Objet et résumé de l'invention

La présente invention a donc pour but de palier les inconvénients présentés par les deux procédés actuellement connus en proposant un procédé de contrôle d'appareils d'analyse biologique délocalisée destiné à être mis en oeuvre in situ à partir d'un appareil dit de contrôle comprenant les étapes définies par la revendication 1. Un tel procédé permet d'automatiser le contrôle qualité des appareils d'analyse biologique délocalisée dans leur environnement d'usage ordinaire à l'aide d'un appareil de contrôle servant pour le contrôle d'une pluralité d'appareils d'analyse délocalisée. Cet appareil de contrôle communique avec un système informatique centralisateur qui est apte à déterminer un biais convenable éventuellement différent pour chaque évènement de contrôle.

En particulier, le biais est déterminé de manière rationnelle en fonction de données qualitatives connues et rationnalisées grâce à une saisie ou une réception de données qualitatives codées sous un format numérique, soit directement par l'opérateur lui-même qui saisi alors des codes, soit par une transformation de la saisie de l'opérateur en un code numérique.

L'utilisation de données qualitatives qualifiant l'environnement de l'appareil d'analyse délocalisée devant être contrôlé permet également de contrôler les appareils d'analyse délocalisée en fonction de l'environnement dans lequel ils sont utilisés. La présence de telles données qualitatives permet également de vérifier périodiquement, non seulement l'état et les performances des appareils d'analyse délocalisée, mais également l'état des consommables utilisés.

Le procédé selon l'invention propose donc de sécuriser l'utilisation des appareils d'analyse délocalisée en permettant un suivi de ces matériels relativement continu et homogène. En effet, il présente l'avantage d'uniformiser les résultats obtenus par les appareils d'analyse délocalisée ou du moins de donner des éléments de comparaison entre eux. La connaissance du biais existant entre les mesures réalisées par des appareils de types différents est un avantage important car, ainsi, la comparaison tient directement compte du biais. En effet, la comparaison utilise alors directement une valeur cible attendue et calculée à partir de la valeur mesurée sur l'appareil de référence et reçue en provenance de l'appareil de contrôle et du biais. Le biais est déterminé et appliqué, selon l'invention, en fonction des situations observées et résumées dans les données qualitatives relatives à l'environnement de l'appareil de biologie délocalisée. On permet ainsi de rendre l'expertise de l'opérateur plus pertinente.

L'expertise du biologiste s'applique alors à la définition de toutes conditions d'environnement et d'utilisation particulières de toute nature pouvant altérer la qualité des résultats obtenus dans le contexte particulier d'utilisation hors laboratoire.

Le rôle du biologiste est de proposer des « questionnaires » paramétrés pour qualifier ces conditions particulières d'utilisation dans l'établissement de soins. Ces conditions sont alors comparées aux recommandations initiales du fabricant des produits utilisés.

Dans un cadre plus général, puisque les mesures effectuées avec les appareils d'analyses délocalisées font l'objet d'un contrôle, le procédé permet de sécuriser les actes effectués en fonction de ces analyses.

En outre, l'invention permet de prendre en compte l'environnement d'utilisation de l'appareil de biologie délocalisée, ce qui est très utile. La variabilité de l'environnement de réalisation des analyses délocalisées peut influer sur les résultats obtenus au point de rendre cette pratique dangereuse. Par exemple, un lecteur de glycémie souillé peut constituer un vecteur d'infection nosocomiale majeur, au-delà des perturbations possibles de ces résultats.

Un relevé détaillé de l'environnement d'utilisation lié aux écarts de résultats obtenus à partir de solutions connues, vise à permettre au biologiste :

- de « recadrer » continuellement les limites d'emploi des appareils délocalisés, y compris lorsque les analyses sont faites à domicile (« home tests » en anglais), dans l'officine du médecin (« doctors tests » en anglais) dans la pratique clinique quotidienne des établissements de soins ou tout autre lieu d'utilisation,
- de compléter la formation des utilisateurs ou de suspendre le cas échéant l'usage de l'appareil, lorsque le constat précis des circonstances d'utilisation des matériels et résultats obtenus, montrent que les limites de la méthode sont atteintes et que les risques deviennent démesurés lorsque les appareils sont utilisés en dehors du cadre « sécurisé » d'un laboratoire d'analyses médicales.

Avec la réception du compte-rendu, l'appareil de contrôle, quand il est bidirectionnel, est alors une plateforme pour suivre le contrôle de la saisie des données jusqu'à l'obtention du résultat.

Dans d'autres réalisations, le compte rendu est envoyé sur un terminal informatique, par exemple situé à proximité de l'appareil de contrôle ou dans un local, par exemple hospitalier, dédié au contrôle centralisé des appareils d'analyse délocalisée. Ce terminal informatique est associé aux appareils de contrôle en tant qu'il reçoit les comptes-rendus relatifs aux appareils d'analyse délocalisée préalablement contrôlés par ces appareils de contrôle.

Selon une réalisation préférentielle de l'invention, l'appareil de contrôle est un appareil d'analyse biologique de référence sur lequel, lors de l'étape d'acquisition, est directement mesurée la valeur de la variable biologique de la solution de contrôle.

Avec une telle caractéristique, l'appareil de référence permettant de réaliser l'analyse de référence pour le contrôle des appareils de biologie délocalisée sert d'appareil de contrôle apte à envoyer les données vers le système centralisateur. Une telle réalisation permet de concentrer les fonctions et de la combiner au sein d'un seul appareil.

Selon une autre caractéristique particulière de l'invention, le procédé comprend une étape d'alerte déclenchée en fonction du contenu du compte-rendu reçu en provenance du système informatique.

Une telle caractéristique permet une automatisation encore plus élevée du contrôle en donnant directement à l'opérateur un avis sur l'état de l'appareil d'analyse délocalisée contrôlé. L'expertise de l'opérateur devient alors un paramètre moins important qu'avec les procédés de contrôle connus. Le compte-rendu envoyé par le système centralisateur comprend alors une donnée d'alerte qui est ensuite interprétée par l'appareil de contrôle ou le terminal informatique qui reçoit ce compte-rendu.

Selon une caractéristique avantageuse, le stockage des données de comparaison associées à des données qualitatives est réalisé sous la forme d'une base de données.

Le stockage des données de comparaison associées à des données qualitatives permet en effet de constituer une base de données qui peut être ultérieurement utilisée pour des évaluations statistiques ou pour des déterminations ultérieures d'un paramètre, par exemple d'un biais correspondant à une situation environnementale particulière.

En outre, l'organisation sous forme de base de données offre des possibilités de traçabilité, de gestion et d'édition de données relatives au contrôle des appareils d'analyse délocalisée.

Plusieurs modes de réalisation du système informatique sont envisageables.

Il peut en effet être constitué d'une seule machine ou d'une pluralité de machines reliées entre elles par un réseau.

Ainsi, le procédé selon l'invention peut fonctionner sous la forme d'une communauté de services réseaux installés au sein d'une pluralité de serveurs.

Selon une caractéristique avantageuse, le procédé selon l'invention est apte à fonctionner en mode initialisation dont le but est de définir un biais pour chaque appareil d'analyse biologique délocalisée en fonction de la solution de contrôle utilisée, le biais étant défini égal à la différence entre la valeur de la variable mesurée avec l'appareil d'analyse biologique délocalisée et la valeur mesurée avec l'appareil de contrôle ou à la moyenne de cette différence sur une pluralité de mesures par l'appareil d'analyse délocalisée et le biais étant stocké en tant que données de comparaison.

Avec une telle caractéristique, les données de comparaison incluent alors, au minimum, une donnée sur le biais attendu en fonction de la situation de contrôle, donc des données qualitatives, et de l'appareil d'analyse délocalisée considéré. Les étapes de détermination de biais ultérieurs utilisent ces données de comparaison stockées. On remarque ici que le biais, défini pour chaque appareil, pourra être en réalité défini pour chaque type d'appareil puis attribué à chaque appareil d'un même type. On remarque également que le biais, qui peut être un facteur de correction, sera défini pour chaque situation particulière de l'utilisation d'un ou plusieurs appareils. Pour définir les biais, on utilise avantageusement des analyses statistiques des résultats de la base de données par groupe de pairs ou par tout type de critères (méthode, appareil, etc) qui définissent une situation particulière.

Selon un mode de réalisation, l'étape de stockage de données de comparaison n'est réalisée qu'en début d'utilisation et de contrôle d'un appareil d'analyse biologique délocalisée.

Une telle caractéristique permet que soit réalisée une initialisation progressive du procédé de contrôle, en particulier par rapport à la détermination du biais. Il s'agit en fait de réaliser une sorte d'apprentissage du procédé ensuite utilisé pour les contrôles suivants.

D'autres moyens pour définir les biais pourront être mises en oeuvre, notamment on pourra définir les biais et valeurs cibles attendues indépendamment du procédé selon l'invention et préalablement à toute utilisation ou contrôle des appareils de biologie délocalisée et de l'appareil de référence. Ces biais et valeurs cibles seront alors stockées dans le système centralisateur préalablement aux contrôles. Bien sur, des révisions des biais ou des facteurs de correction et des valeurs cibles doivent avoir lieu ultérieurement et régulièrement pour éviter toutes dérives et déceler toutes anomalies.

Selon une caractéristique particulière de l'invention, les données qualitatives incluent des données qualifiant la solution de contrôle.

En effet, la solution de contrôle peut être choisie parmi les types de solutions suivants : une solution fournie par le fabricant, un sang du patient, un sang total du commerce, une solution de contrôle dite « universelle ». Dans ce cadre, le terme « universelle » signifie que la mesure de la grandeur biologique analysée est sensiblement la même quel que soit l'appareil d'analyse biologique utilisée.

Une telle donnée qualitative permet de définir un biais pour chaque solution de contrôle. Dans ce cas, lors de l'étape de détermination du biais, et dans le cas où le paramètre « solution de contrôle » est le seul paramètre à utiliser pour la détermination du biais, seule la connaissance de la donnée qualitative relative à la solution de contrôle permet de déterminer le biais. On comprend bien d'ailleurs que la valeur du biais sera forcément différente selon que des solutions de contrôle différentes seront utilisées. L'indication de données sur la solution de contrôle au sein des données qualitatives permet donc, en particulier, de déterminer le biais de manière plus exacte.

Il est, bien sur, envisagé que le paramètre relatif à la solution de contrôle soit utilisé en combinaison avec d'autres paramètres entrant dans la détermination du biais, par exemple le type d'appareil, la température, l'hygrométrie, etc.

En fonction des applications, la variable biologique est choisie parmi les variables suivantes : glycémie, gaz du sang, hématocrite, lactates, ionogramme sanguin ou urinaire, glucose urinaire, créatinine, hémoglobineA1c, acide urique, cholestérol, triglycérides ou tout autre paramètre biologique, même inconnu à ce jour, dès lors qu'on dispose, pour faire cette mesure, d'appareils d'analyse biologique délocalisée et d'un appareil de contrôle.

L'invention concerne également un appareil de contrôle, à partir duquel un procédé selon l'invention est mis en oeuvre.

Cet appareil d'analyse biologique de contrôle comprend pour cela :

- des moyens de saisie ou de réception de données qualitatives caractérisant un appareil de biologie délocalisée devant être contrôlé et caractérisant l'environnement dans lequel se trouve cet appareil,
- des moyens de saisie ou de réception de la valeur d'une variable biologique d'une solution dite de contrôle mesurée par l'appareil de biologie délocalisée devant être contrôlé,
- des moyens d'acquisition de la valeur de la variable biologique de la solution de contrôle mesurée par un appareil d'analyse biologique de référence,
- des moyens de transmission de la valeur saisie ou reçue, de la valeur mesurée avec l'appareil de contrôle et des données qualitatives saisies ou reçues vers un système informatique centralisateur apte à déterminer un biais attendu entre la valeur de la variable biologique saisie ou reçue et la valeur mesurée avec l'appareil de référence en fonction des données qualitatives saisies ou reçues, dont celles renseignant l'environnement dans lequel l'appareil est utilisé, et de données de comparaison stockées dans le système informatique, à comparer la valeur mesurée sur l'appareil de biologie délocalisée avec la valeur mesurée par l'appareil de référence en tenant compte du biais, à stocker des données de comparaison relatives à la comparaison effectuée associées à des données qualitatives, à élaborer un compte rendu de contrôle et à l'envoyer vers l'appareil de contrôle ou un terminal informatique associé à cet appareil de contrôle.

Selon un mode de réalisation particulier, l'appareil d'analyse biologique de contrôle comprend en outre des moyens de réception du compte rendu de contrôle.

Selon une autre caractéristique particulière de l'invention, l'appareil d'analyse biologique de contrôle comprend en outre des moyens d'alerte déclenchés en fonction du contenu du compte rendu.

L'invention concerne aussi un système informatique centralisateur apte à communiquer avec une pluralité d'appareils d'analyse biologique de contrôle selon l'invention.

Le système informatique centralisateur comprend pour cela :

- des moyens pour recevoir la valeur saisie ou reçue par l'appareil de contrôle, la valeur mesurée avec un appareil de référence et les données qualitatives transmis par l'appareil de contrôle,
- des moyens de détermination d'un biais attendu entre la valeur de la variable biologique saisie après mesure par l'appareil de biologie délocalisée et la valeur mesurée avec l'appareil de référence en fonction des données qualitatives saisies et de données de comparaison stockées dans le système informatique,
- des moyens de comparaison de la valeur mesurée sur l'appareil de biologie délocalisée avec la valeur mesurée par l'appareil de référence en tenant compte du biais,
- des moyens de stockage pour stocker des données de comparaison relatives à la comparaison effectuée,
- des moyens pour élaborer un compte rendu de contrôle,
- des moyens de transmission pour envoyer le compte rendu de contrôle vers l'appareil de contrôle ou vers un terminal informatique associé à cet appareil de contrôle.

Selon une implémentation préférée, les différentes étapes du procédé sont déterminées par des instructions de programmes d'ordinateurs.

En conséquence, l'invention vise aussi un programme d'ordinateur sur un support d'informations, ce programme étant susceptible d'être mis en oeuvre dans un appareil de contrôle apte à communiquer avec un système informatique centralisateur selon l'invention, ce programme comportant des instructions adaptées à la mise en oeuvre des étapes suivantes :

- saisie ou de réception de données qualitatives caractérisant un appareil de biologie délocalisée devant être contrôlé,
- saisie ou de réception de la valeur d'une variable biologique d'une solution dite de contrôle mesurée par l'appareil de biologie délocalisée devant être contrôlé,
- acquisition de la valeur de la variable biologique de la solution de contrôle mesurée par un appareil d'analyse biologique de référence,
- transmission de la valeur saisie, de la valeur mesurée et des données qualitatives saisies vers un système informatique centralisateur apte à déterminer un biais attendu entre la valeur de la variable biologique saisie après mesure par l'appareil de biologie délocalisée et la valeur mesurée avec l'appareil de référence en fonction des données qualitatives saisies et de données de comparaison stockées dans le système informatique, à comparer la valeur mesurée sur l'appareil de biologie délocalisée avec la valeur mesurée par l'appareil de contrôle en tenant compte du biais, à stocker des données de comparaison relatives à la comparaison effectuée associées à des données qualitatives, à élaborer un compte rendu de contrôle et à l'envoyer vers l'appareil d'analyse biologique de contrôle ou vers un terminal informatique.

L'invention concerne aussi un programme d'ordinateur sur un support d'informations, ce programme étant susceptible d'être mis en oeuvre dans un système informatique centralisateur apte à communiquer avec un appareil de contrôle selon l'invention, ce programme comportant des instructions adaptées à la mise en oeuvre des étapes suivantes :

- recevoir la valeur saisie, la valeur mesurée et les données qualitatives transmis par l'appareil de contrôle,
- déterminer un biais attendu entre la valeur de la variable biologique saisie après mesure par l'appareil de biologie délocalisée et la valeur mesurée avec l'appareil de contrôle en fonction des données qualitatives saisies et de données de comparaison stockées dans le système informatique,
- comparaison de la valeur mesurée sur l'appareil de biologie délocalisée avec la valeur mesurée par l'appareil de contrôle en tenant compte du biais,
- stockage pour stocker des données de comparaison relatives à la comparaison effectuée,
- élaboration d'un compte rendu de contrôle,
- transmission du compte rendu de contrôle vers l'appareil d'analyse biologique de contrôle ou vers un terminal informatique.

Ces programmes peuvent utiliser n'importe quel langage de programmation, et être sous la forme de code source, code objet, ou de code intermédiaire entre code source et code objet, tel que dans une forme partiellement compilée, ou dans n'importe quelle autre forme souhaitable, par exemple un code html.

L'invention vise aussi un support d'informations lisible par un appareil de contrôle ou un système informatique, et comportant des instructions d'un programme d'ordinateur tel que mentionné ci-dessus.

Le support d'informations peut être n'importe quelle entité ou dispositif capable de stocker le programme. Par exemple, le support peut comporter un moyen de stockage, tel qu'une ROM, par exemple un CD ROM ou une ROM de circuit microélectronique, ou encore un moyen d'enregistrement magnétique, par exemple une disquette (floppy disc) ou un disque dur, un moyen d'enregistrement optique ou magnéto-optique, une RAM, une mémoire Flash ou tout support de flux ou de stockage de données numérisées ou non.

D'autre part, le support d'informations peut être un support transmissible tel qu'un signal électrique ou optique, qui peut être acheminé via un câble électrique ou optique, par radio ou par d'autres moyens. Le programme selon l'invention peut être en particulier téléchargé sur un réseau de type Internet.

Alternativement, le support d'informations peut être un circuit intégré dans lequel le programme est incorporé, le circuit étant adapté pour exécuter ou pour être utilisé dans l'exécution du procédé en question.

Brève description des dessins

D'autres caractéristiques et avantages de la présente invention ressortiront de la description faite ci-dessous, en référence aux dessins annexés qui en illustrent un exemple de réalisation dépourvu de tout caractère limitatif. Sur les figures :

- la figure 1 représente schématiquement les moyens pour mettre en oeuvre un procédé selon l'invention ;
- la figure 2 est un organigramme schématique d'un procédé selon l'invention ;
- la figure 3 illustre schématiquement la présence d'un biais entre les mesures réalisées avec les appareils d'analyse délocalisée et l'appareil de contrôle ;
- la figure 4 représente schématiquement un appareil de contrôle selon l'invention.

Description détaillée d'un mode de réalisation

La figure la représente schématiquement la mise en oeuvre d'un procédé selon une première réalisation de l'invention pour contrôler un ensemble d'appareils 10, 11, 12, 13, 14 d'analyse biologique délocalisée. Ces appareils 10, 11, 12, 13 et 14 sont aptes à mesurer une grandeur biologique et à donner une valeur de cette grandeur. Par exemple, dans la suite, on considérera que les appareils 10, 11, 12, 13 et 14 sont des appareils de mesure de glycémie destinés à être placés auprès de patients, par exemple dans plusieurs chambres d'hôpital ou de maison médicalisée.

L'invention ne se limite néanmoins pas aux appareils placés au chevet du patient, notamment dans un hôpital. Elle concerne également les appareils ambulatoires placés chez le patient et en tout lieu en dehors du laboratoire, notamment dans les véhicules d'urgence (SAMU), dans les locaux de dispensaires, de cliniques, d'officines etc. On parle souvent, dans ce contexte, de « home tests » et de « doctors tests » en anglais.

L'invention ne se limite pas non plus aux appareils de mesure de glycémie mais concerne tout appareil de mesure en dehors du laboratoire par toutes techniques seules ou combinées de mesures chimiques, physiques, physicochimiques, optiques, électroniques, informatiques, automatiques ou par des procédés nouveaux ayant pour objet une analyse biologique.

**EP 2 095 287 B1**

Selon le procédé de l'invention, une première mesure QTD[i] de glycémie d'une solution de contrôle SC est réalisée avec chaque appareil d'analyse biologique délocalisée i avec i = 10, 11, 12, 13 ou 14.

La solution de contrôle SC peut être un sang total obtenu par prélèvement capillaire ou par prélèvement veineux, une solution propriétaire d'un fabricant d'appareil ou encore être une solution développée spécialement pour le contrôle de fiabilité des appareils de biologie délocalisée. En tout cas, la solution de contrôle SC doit être identique tout le long du procédé de contrôle de fiabilité des appareils d'analyse délocalisée. On sait en outre que le terme « solution de contrôle » peut couvrir en réalité l'usage de plusieurs solutions, chacune d'un niveau différent de la variable mesurée. Trois solutions de niveau bas, moyen et haut pourront être ainsi utilisées en tant que « solution de contrôle » SC. Les principes de l'invention restent néanmoins identiques. Dans ce dernier exemple, chaque donnée quantitative correspond alors à un triplet de valeurs.

Les valeurs mesurées QTD[i] par chacun des appareils d'analyse délocalisée sont par exemple saisies sur un assistant numérique personnel (acronyme anglais : «PDA») 2, appareil de contrôle dans cette réalisation. Un appareil d'analyse biologique dit de référence 3 réalise aussi une mesure de la valeur de la variable biologique. L'appareil de référence 3 peut aussi être nommé appareil de comparaison. L'appareil de contrôle 2 est alors apte à acquérir cette valeur mesurée sur l'appareil de référence. Cette acquisition est ici avantageusement réalisée par saisie ou réception de la valeur mesurée sur l'assistant numérique personnel 2. L'assistant numérique personnel 2 est aussi apte à transmettre des données à un système centralisateur 4 par l'intermédiaire d'un terminal informatique installé dans le laboratoire ou le service dans lequel les appareils de biologie délocalisée sont contrôlés. Cette transmission inclura des données qualitatives QLD[i] également saisies sur l'assistant numérique personnel 2. Ces étapes sont semblables à celles présentées dans la réalisation préférentielle dont une description schématique est présentée dans la suite.

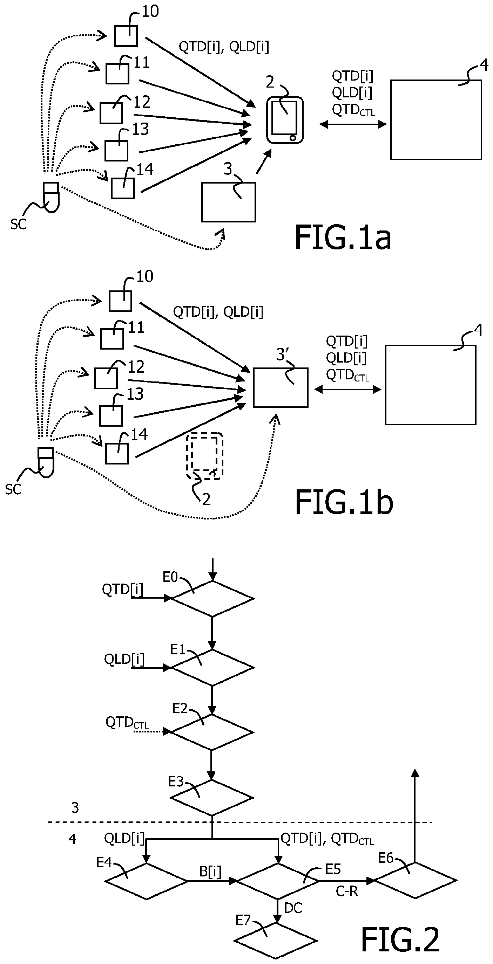

La figure 1b représente schématiquement la mise en oeuvre d'un procédé selon une réalisation préférentielle de l'invention pour contrôler un ensemble d'appareils 10, 11, 12, 13, 14 d'analyse biologique délocalisée. Ces appareils 10, 11, 12, 13 et 14 sont aptes à mesurer une grandeur biologique et à donner une valeur de cette grandeur. Ici encore, par exemple, les appareils 10, 11, 12, 13 et 14 sont des appareils de mesure de glycémie destinés à être placés auprès de patients, par exemple dans plusieurs chambres d'hôpital ou de maison médicalisée.

Selon le procédé de l'invention, une première mesure QTD[i] de glycémie d'une solution de contrôle SC est réalisée avec chaque appareil d'analyse biologique délocalisée i, i = 10, 11, 12, 13 ou 14.

La figure 2 représente schématiquement un procédé selon l'invention.

Les valeurs mesurées QTD[i] par chacun des appareils d'analyse délocalisée sont par exemple saisies sur un assistant numérique personnel 2 afin d'être transmise ensuite à un appareil d'analyse biologique dit de contrôle 3' dans une étape E0. Une telle transmission peut être réalisée à l'aide d'une connexion avec ou sans fil selon une technologie choisie parmi celles connues pour le transfert de données d'un appareil électronique à un autre.

Il est également envisageable de mettre en oeuvre l'invention sans utiliser d'assistant numérique personnel 2 qui est d'ailleurs représenté en pointillés. Dans ce cas, les appareils d'analyse délocalisée i peuvent comprendre directement eux-mêmes des moyens pour transmettre la valeur mesurée QTD[i] à l'appareil de contrôle 3'. L'appareil de contrôle 3' peut aussi disposer de moyens de saisie pour que soient saisies directement des données parmi lesquelles les valeurs mesurées QTD[i] sur les appareils d'analyse biologique délocalisée i.

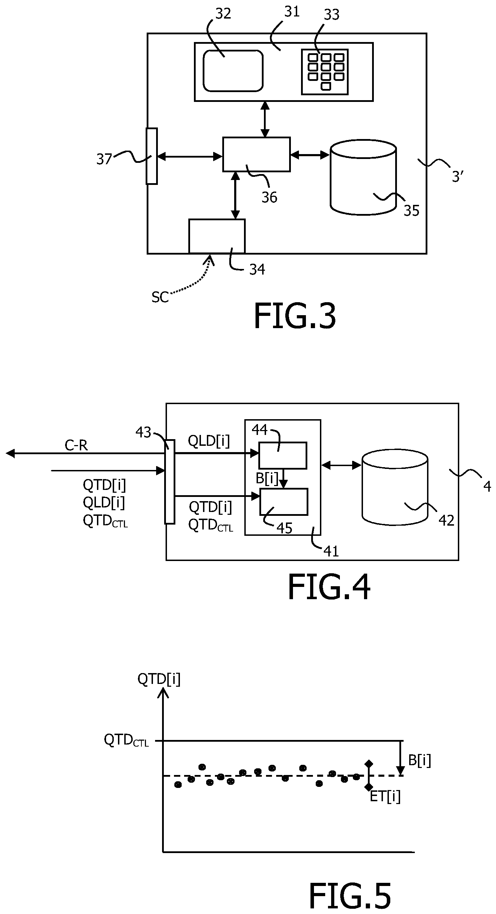

La figure 3 représente schématiquement un tel dernier mode de réalisation pour un appareil de contrôle 3' selon l'invention. Dans ce mode de réalisation, l'appareil de contrôle 3' inclut une interface homme machine 31 comprenant au moins des moyens d'affichage constitués par un écran 32 et des moyens de saisie qui sont constitués par un clavier 33.

L'étape E0 est donc une étape de saisie ou de transmission des valeurs de la variable biologique telles que mesurées sur les appareils d'analyse délocalisée i.

Selon le procédé de l'invention, dans une étape E1, des données qualitatives QLD[i] sont également fournies préalablement, successivement ou simultanément à la transmission ou à la saisie des valeurs mesurées sur les appareils d'analyse délocalisée i. On utilise, pour ces données qualitatives, les mêmes moyens de transmission ou de saisie que pour les données quantitatives. Ces données qualitatives QLD[i] identifient au minimum chacun des appareils de biologie délocalisée i devant être contrôlés. Ainsi, au moins une donnée qualitative d'identification de l'appareil d'analyse délocalisée i accompagne chaque valeur QTD[i] de la variable biologique saisie ou transmise.

Des exemples d'autres types de données qualitatives et de codage de ces données sont donnés dans la suite.

Ensuite, selon la réalisation préférentielle, une étape E2 de mesure de la valeur $QTD_{CTL}$ de la variable biologique au sein de la solution de contrôle SC est réalisée à l'aide de l'appareil de contrôle 3' qui est précisément aussi l'appareil de référence.

Pour cela, comme représenté schématiquement sur la figure 3, l'appareil de contrôle 3' comprend des moyens de mesure 34. Ces moyens de mesure 34 fonctionnent, par exemple, à l'aide de supports réactifs de mesure, par exemple des bandelettes ou des cuvettes sur ou dans lesquelles on dépose un échantillon du fluide à analyser, ici, la solution de contrôle SC. L'appareil de contrôle est alors globalement constitué de moyens de mesure, par exemple un photomètre ou encore un analyseur à capteurs analytiques, associé à un pavé numérique pour la saisie de données.

L'appareil de contrôle 3' dispose alors des valeurs QTD[i] saisies ou reçues telles que mesurées par les appareils de biologie délocalisée, de la valeur mesurée $QTD_{CTL}$ par l'appareil de contrôle et de données qualitatives QLD[i] saisies ou reçues. Ces données sont alors par exemple stockées dans une mémoire 35 gérée par un microprocesseur 36 au sein de l'appareil de contrôle 3' représenté sur la figure 3.

L'appareil de contrôle 3' comprend encore des moyens de transmission/réception 37 aptes à procéder à une étape E3 de transmission de ces données vers un système informatique centralisateur 4. Ce système 4 peut être réalisé sur une seule machine ou sur une pluralité de machines organisées en réseau. La communication entre l'appareil de contrôle 3' et le système centralisateur 4 peut être unidirectionnelle ou bidirectionnelle.

Comme représenté sur la figure 4, le système informatique 4 comprend des moyens de transmission/réception de données 43, au moins des moyens de traitement de données 41 et des moyens de stockage 42 des données reçues. Les moyens de stockage 42 sont avantageusement organisés sous forme d'une base de données dont les champs contiennent des données qualitatives et des données quantitatives.

Dans un mode de réalisation de l'invention, les appareils d'analyse délocalisée sont enregistrés auprès du système informatique 4 préalablement à tout contrôle. Cet enregistrement peut se réaliser automatiquement lors d'un abonnement demandé par l'appareil de contrôle 3' apte à les contrôler. L'implémentation de l'invention peut donc être réalisée sous la forme d'un abonnement à un service réseau accessible à partir d'appareils de contrôle, que celui-ci soit un appareil de contrôle selon la première réalisation ou la réalisation préférentielle. Soit un terminal informatique local sur lequel l'assistant numérique personnel 2 transmet les données stockées, soit l'appareil de contrôle 3' lui-même comprennent donc les moyens matériels et logiciels pour communiquer alors avec les moyens logiciels installés dans le système centralisateur 4 auquel il est abonné. Cet abonnement donne l'accès à la base de données stockée dans le système centralisateur 4.

C'est à partir de cette base de données qu'est réalisée l'exploitation statistique, par les moyens de traitement 41, des données qualitatives et quantitatives reçues en provenance de l' (ou des) appareil(s) de contrôle 3'.

En effet, le système informatique centralisateur 4 peut être en relation avec une pluralité d'appareils de contrôle situé par exemple dans une pluralité de services d'une même structure hospitalière.

Les données stockées dans les moyens de stockage 42 sont, en particulier, utilisées par les moyens de traitement 41 pour évaluer l'exactitude des mesures réalisées par les différents appareils d'analyse biologique délocalisée i.

Cette exactitude des mesures est avantageusement évaluée du point de vue de la reproductibilité et de la précision.

Pour contrôler la reproductibilité, il est utile de connaître un intervalle de valeur dans lequel sont attendues les valeurs mesurées QTD[i]. Cet intervalle, centré sur une valeur moyenne, est typiquement défini à l'aide d'un écart maximum à la moyenne ET[i].

Un tel écart maximum à la moyenne ET[i] est généralement fonction du type d'appareil de biologie délocalisée i utilisé et commun à tous les appareils de biologie délocalisée d'un type donné.

Afin de réaliser un contrôle de précision, il est nécessaire de connaître en outre un biais B[i] éventuel existant entre la valeur mesurée avec chaque appareil de biologie délocalisée i et la valeur mesurée $QTD_{CTL}$ sur l'appareil de contrôle 3'. Le biais B[i] est une valeur exprimée en valeur absolue, et donc positive. Le biais B[i] peut être ajouté ou retranché à la valeur mesurée $QTD_{CTL}$ sur l'appareil de contrôle 3'.

Le terme « biais » désigne une valeur numérique ou une fonction mathématique ou un ensemble de fonctions mathématiques, calculées ou décrites qui, appliquées aux valeurs mesurées permet d'écrire une conclusion de contrôle finale relative au matériel contrôlé.

Cette valeur numérique ou cette fonction mathématique ou cet ensemble de fonctions mathématiques, seules ou combinées entre elles, sont un facteur de correction.

Un tel facteur de correction sert en particulier pour faire une comparaison entre une mesure sur sang total et une mesure sur plasma, une mesure d'un élément à une température et une mesure d'un élément à une température différente ou à tout autre différentiel de conditions environnementales comme l'hygrométrie, la pression atmosphérique, etc.

En tout cas, la prise en compte du biais ou facteur de correction issu de tout procédé de calcul lors des comparaisons permet une conclusion directement utilisable par le destinataire d'un compte-rendu de contrôle sans qu'il ait à tenir compte, par lui-même, d'un éventuel décalage entre les mesures. Le biais permet, au sens de l'invention, de rendre comparable deux données distinctes. En outre, le biais ou facteur de correction peut prendre en compte un changement d'unités entre deux données.

Le biais B[i] est avantageusement défini comme la différence entre une unique valeur QTD[i] ou une moyenne de valeurs QTD[i] observée(s) lors d'une série de mesures d'un échantillon unique de solution de contrôle SC et la valeur vraie VV de cet échantillon. La valeur vraie VV peut être définie dans l'absolu lorsqu'il s'agit d'une solution de fabricant ou de manière relative lorsqu'elle est mesurée sur l'appareil de contrôle ou encore sur un appareil de laboratoire.

Dans ce dernier cas, un biais est avantageusement aussi défini pour l'appareil de contrôle en utilisant une moyenne des valeurs QTD$_{CTL}$ obtenues pour une série de mesures.

D'une manière générale, dans le cas où une moyenne de valeur QTD[i] est utilisée, le biais peut être exprimé en pourcentage selon la formule suivante :

$$\text{Biais en \%} = 100 * (\text{Moyenne QTD[i]} - VV) / VV.$$

Il peut aussi être directement calculé en valeur relative :

$$\text{Biais relatif} = \text{Moyenne QTD [i]} - VV.$$

On remarque ici que ces biais en pourcentage et relatif peuvent prendre des valeurs négatives. Cela se reflète en réalité par une soustraction ou une addition du biais défini comme étant la valeur absolue du biais, à la valeur cible. Bien entendu, cette soustraction ou addition peut être précédée d'une multiplication dans le cas d'un biais exprimé en pourcentage. La valeur cible peut être la valeur vraie VV quand celle-ci est définie grâce à l'appareil de contrôle 3'.

Le biais sera avantageusement redéfini à chaque changement de lot de supports de réaction, à chaque changement de réactif, à chaque changement de solution de contrôle, à chaque calibration, à chaque changement d'appareil ou type d'appareil.

La connaissance d'un tel biais B[i] permet alors d'automatiser le contrôle des appareils d'analyse délocalisée i puisque les moyens de traitement 41 sont rendus capables de comparer les valeurs mesurées QTD$_{CTL}$ par l'appareil de contrôle 3 et par chacun des appareils d'analyse délocalisée i.

On pourra alors détecter par exemple des calibrations inexactes, une maintenance inappropriée ainsi que des utilisations erronées des réactifs ou encore une mauvaise conservation des réactifs, une mauvaise utilisation ou un mauvais paramétrage des appareils.

Si le fonctionnement de l'appareil d'analyse délocalisée est correct, les valeurs mesurées QTD[i] devront alors toujours se situer autour d'une valeur cible calculée à l'aide du biais B[i] qui peut être exprimé en pourcentage ou en valeur de correction d'une valeur de référence. La largeur de l'intervalle dans lequel elles se trouveront autour de la valeur cible est définie par l'écart ET[i].

Outre le fait que les biais B[i] changent en fonction de l'appareil d'analyse délocalisée i contrôlé, les biais B[i] observés sont aussi susceptibles de varier en fonction de la solution de contrôle utilisée pour réaliser le contrôle de fiabilité des appareils de biologie délocalisée i ou encore en fonction des conditions d'utilisation des appareils de biologie délocalisée i et des appareils de contrôle 3 (température, environnement, date du dernier étalonnage...). Ainsi le biais finalement appliqué est le reflet soit d'un cumul d'un biais tel que défini entre l'appareil de référence et l'appareil de biologie délocalisée avec un biais d'environnement local d'utilisation relevé sur place et renseigné au sein des données qualitatives, soit d'un cumul d'un biais tel que défini selon les différences de solution analysée avec l'appareil de biologie délocalisée et l'appareil de référence (par exemple, sang du patient, capillaire et plasma issu du même patient, analysé en laboratoire) et d'un biais d'environnement local d'utilisation relevé sur place et renseigné au sein des données qualitatives. Le biais peut aussi être issu d'un calcul utilisant des fonctions spécifiques.

La connaissance de l'existence d'un tel biais B[i] et sa détermination sont des éléments cruciaux pour l'utilisation des appareils d'analyse délocalisée i concernés. Il est aussi crucial de connaître ses variations en fonction de données qualitatives QLD[i] sur l'environnement de l'appareil d'analyse délocalisée i.

La figure 5 illustre l'existence d'un biais B[i] entre les mesures QTD[i] réalisées sur un appareil de biologie délocalisée i et celle QTD$_{CTL}$ réalisée par un appareil de contrôle 3. Afin que le fonctionnement de l'appareil d'analyse délocalisée puisse être déclaré correct, les mesures QTD[i] doivent se situer dans un intervalle de la largeur de l'écart ET[i] autour d'une valeur cible obtenue en retranchant le biais B[i] à la valeur QTD$_{CTL}$ ou, le cas échéant, en appliquant le pourcentage correspondant au biais à la valeur QTD$_{CTL}$.

L'invention se propose, en fait, de rationnaliser la présence du biais B[i] et de rendre cette présence transparente pour l'utilisateur de l'appareil de contrôle 3.

Jusqu'à présent, un opérateur réalisant un contrôle sur un appareil de biologie délocalisée i utilise généralement des biais prédéfinis et approximatifs. Cela est préjudiciable pour la précision du contrôle voire pour sa fiabilité.

Selon l'invention, le biais B[i] est avantageusement déterminé dans une étape d'initialisation préalable à la mise en oeuvre du procédé avec une solution de contrôle SC donnée.

Au cours de cette initialisation, au moins deux valeurs QTD[i] et QTD$_{CTL}$ sont mesurées pour la solution de contrôle SC, une avec l'appareil de biologie délocalisée i et l'autre avec l'appareil de contrôle 3.

Ces valeurs sont, respectivement, saisie ou reçue et acquise au sein de l'appareil de contrôle 3 ou 3'. Des données qualitatives QLD[i] sont associées à chaque valeur QTD[i] saisie ou reçue. Les données qualitatives QLD[i] comprennent au moins des données d'identification de l'appareil de biologie délocalisée i concerné par le biais B[i] faisant l'objet de l'initialisation.

Le biais B[i] est alors défini, pour l'appareil de biologie délocalisée i, au sein du système centralisateur 4, comme étant la différence entre les deux valeurs QTD[i] et $QTD_{CTL}$.

Une fois ce biais B[i] défini, à l'aide de l'écart maximum à la moyenne ET[i] connu pour le type d'appareil auquel appartient l'appareil de biologie délocalisée i, on définit une fourchette dans laquelle devront se situer les valeurs mesurées QTD[i].

Avantageusement, cette opération d'initialisation comprend la répétition d'un certain nombre de mesures réalisées par l'appareil de biologie délocalisée i, voire de plusieurs mesures réalisées par l'appareil de contrôle 3.

Ainsi le biais B[i] est alors défini comme étant une moyenne des différences observées. La valeur du biais B[i] est alors plus fiable et précise.

Un apprentissage de la valeur du biais B[i] peut aussi être réalisé au cours d'un certain nombre de premiers contrôles de chaque appareil de biologie délocalisé i, quand on sait que l'appareil i est dans des conditions optimales d'utilisation.

Un tel apprentissage peut néanmoins nuire à la détection d'un problème précoce sur l'appareil de biologie délocalisée i. Une dérive de la valeur du biais B[i] défini peut en effet être alors observée sans qu'il ne soit signalé des mesures aberrantes.

Une fois le biais B[i] défini, au sein des moyens de traitement 41, pour chacun des appareils d'analyse délocalisée i et, le cas échéant, pour des données qualitatives distinctes, à chaque réception de données en provenance d'un des appareils d'analyse délocalisée i, il faut déterminer ce biais B[i] en fonction des données qualitatives correspondantes.

Afin de réaliser une telle étape E4 de détermination de ce biais B[i], les moyens de traitement 41 utilisent des moyens de détermination 44 d'un biais B[i] pour chacun des appareils de biologie délocalisée i.

Dans l'étape E4, les moyens de détermination 44 du biais B[i] déterminent le biais, au moins en fonction des données qualitatives QLD[i], en particulier en fonction de celle identifiant l'appareil de biologie délocalisée i contrôlé.

Il est aussi envisagé selon l'invention que les moyens de détermination 44 du biais B[i] inclus dans les moyens de traitement 41 soient en mesure de faire appel à une loi de variation du biais B[i] en fonction de la température ou en fonction d'autres données qualitatives QLD[i] sur l'environnement, par exemple l'hygrométrie ou la pression atmosphérique. A ce niveau là, on peut prévoir que des températures trop élevées (ou, par exemple, une hygrométrie ou une pression atmosphérique trop élevée ou trop basse), signalées parmi les données qualitatives relatives à l'environnement de l'appareil de biologie délocalisée, déclenchent l'absence de fourniture d'un biais et le retour d'un signal de mise en garde dans le compte-rendu sur l'utilisation de l'appareil de biologie délocalisée.

Dans ce cas, les données qualitatives QLD[i] comprendront avantageusement une donnée concernant la température, l'hygrométrie ou la pression atmosphérique par exemple, à laquelle est utilisé l'appareil de biologie délocalisée i. Les données qualitatives QLD[i] incluent donc des données métrologiques et donc quantifiables. Le terme « qualitatives » est donné de manière à distinguer ces données des données « quantitatives » qui sont, elles, relatives aux mesures d'analyse biologique effectuées par l'appareil.

En l'absence d'une telle donnée, une température de référence prédéfinie sera alors utilisée pour déterminer le biais B[i].

Ensuite, dans une étape de comparaison E5, la valeur mesurée QTD[i] transmise à l'appareil de contrôle 3 puis au système centralisateur 4 est comparée à la valeur mesurée $QTD_{CTL}$ par l'appareil de contrôle 3 en tenant compte du biais B[i] déterminé préalablement à cette comparaison dans l'étape E4.

Cette étape de comparaison E5 est réalisée par des moyens de comparaison 45. La comparaison déclenche, au sein des moyens de traitement 41, la création puis l'émission, dans une étape E6, d'un compte rendu C-R à destination de l'appareil de contrôle 3 qui le reçoit grâce aux moyens de transmission/réception 37, lorsque l'appareil de contrôle 3 est bidirectionnel, ou d'un dispositif associé à cet appareil de contrôle 3. Ce dispositif associé peut être un terminal informatique ou tout terminal de réception d'information lié au destinataire tel que fax etc, situé sur le lieu de l'appareil de contrôle 3 ou à proximité de celui-ci ou encore dans le service hospitalier concerné par les contrôles.

Le compte rendu C-R contient une restitution d'au moins une partie des données qualitatives et la mise en évidence de résultats pertinents. Ces résultats pertinents peuvent être résumés par une simple mention « bon », « à vérifier », « à changer » en fonction de l'écart de la valeur mesurée par rapport à une valeur attendue en tenant compte du biais B[i]. Dans le cas où il faut vérifier l'appareil d'analyse délocalisée i, une mesure effectuée avec cet appareil d'analyse délocalisée i peut alors toujours être comparée à une mesure effectuée par un dosage sur sang veineux effectué en urgence dans un laboratoire et être soumise à l'expertise d'un biologiste.

Le compte rendu contient par exemple une date, une identification de l'appareil d'analyse délocalisée, dit lecteur, un code sur le service dans lequel l'appareil est installé, la fréquence d'utilisation, l'état du lecteur, la régularité ou non d'un contrôle hebdomadaire, l'état de la solution de contrôle, la présence ou non d'une brochure de procédure d'utilisation de l'appareil de biologie délocalisée, le numéro de lot des bandelettes utilisées, l'état des bandelettes, la qualité de la calibration du lecteur, le pourcentage d'écart à la valeur cible. Il peut aussi comprendre des informations sur la péremption des électrodes, la présence de l'électrode de calibration. Ce pourcentage d'écart est directement utilisé pour décider si l'appareil est en état correct, à vérifier ou à changer.

La comparaison déclenche aussi une étape de stockage E7 de données de comparaison DC dont font, par exemple, partie la différence entre les valeurs mesurées QTD[i] par l'appareil de biologie délocalisée i et celle QTD$_{CTL}$ mesurée par l'appareil de contrôle 3 quand le biais B[i] est déterminé par un apprentissage lors des premiers contrôles d'un appareil de biologie délocalisée i.

Les données de comparaison DC constituent alors une base de données utile pour faire un suivi global d'un parc d'appareils de biologie délocalisée, y compris un suivi statistique des problèmes observés.

Néanmoins, le biais peut aussi être défini préalablement à tout contrôle et en dehors de toute initialisation du procédé. Dans ce cas, les valeurs de biais, écarts et d'autres paramètres de contrôle sont définies en dehors de l'utilisation d'un quelconque appareil de contrôle et saisies au sein du système centralisateur afin d'être utilisées ultérieurement. Des mesures préalables en laboratoire complétées de calculs statistiques peuvent en particulier être ici utilisés et ce, pour chacune des solutions de contrôle envisagées.

Un couplage est réalisé entre les différentes données qualitatives QLD[i] et ces données de comparaison DC afin de déterminer quels paramètres qualitatifs influent sur le fonctionnement et l'état des appareils de biologie délocalisée i.

Les données stockées peuvent aussi être utilisées comme une base d'archives.

En utilisant une telle base d'archive, il est possible de réaliser des contrôles des appareils d'analyse délocalisée en utilisant un sang de patient. Dans un tel cas, la base d'archive est utilisée pour savoir statistiquement quel biais doit être attendu en fonction des données qualitatives saisies. Le biais est alors notamment déterminé en fonction de caractéristiques du patient incluses dans les données qualitatives.

Les données qualitatives comprendront alors avantageusement l'âge du patient. Par exemple, le biais observé pour un sang de nouveau-né dans les mêmes conditions environnementales pourra être utilisé pour le contrôle d'un des appareils d'analyse délocalisée à l'aide d'un sang de nouveau-né.

Une information sur le type de service (néo-natal notamment) peut aussi être utilisée afin qu'une moyenne du biais attendu puisse être déterminé en fonction de l'âge, au moins approximatif, du patient.

La détermination du biais peut être aussi fonction du taux bas ou élevé du composant mesuré. L'utilisation de la base de données trouve alors tout son sens et toute son utilité.

En outre, la base de données étant généralement en dehors des structures de santé ou du moins dans un lieu dédié elle présente des garanties d'anonymat et de sécurisation quant à l'exactitude des données.

Les données qualitatives peuvent aussi donner des informations sur l'hémoconcentration ou l'hémodilution.

Les données qualitatives peuvent être descriptives des produits employés et de l'environnement d'utilisation de l'appareil d'analyse délocalisée.

Elles comprennent avantageusement des informations sur le type de l'appareil d'analyse délocalisée, l'état de l'appareil, de l'entretien de l'appareil, la méthode d'emploi, la localisation, le numéro de série de l'appareil, la fréquence d'utilisation, la calibration. Elles comprennent aussi avantageusement des données horodatées. Les données d'identification peuvent être saisies ou être détectée automatiquement par un assistant numérique personnel ou encore directement par l'appareil de contrôle.

Ces données qualitatives sont avantageusement saisies selon un codage numérique. Elles sont alors qualifiées à l'aide d'un questionnaire séquentiel reprenant chacune des données d'environnement d'utilisation de l'appareil d'analyse délocalisée contrôlé et associant un code, par exemple un chiffre, à chaque état de chaque donnée.

Dans les données qualitatives ainsi saisies, on retrouve les données utiles pour réaliser le compte rendu et pour réaliser des études statistiques sur le fonctionnement des appareils d'analyse délocalisée.

Les données qualitatives peuvent par exemple être codées sur 20 chiffres de la façon suivante :

1. Code service (2 chiffres choisis dans une liste)

2. N° série du lecteur (3 chiffres choisis dans une liste de numéros d'équipement des lecteurs).

3. Fréquence d'utilisation (1 chiffre) :

0 : moins d'une fois par jour

1 : 1 à 4 fois par jour

2 : 4 à 20 fois par jour

3 : Plus de 20 fois par jour

4 : Réservé chambre patient

9 : Chambre en isolement

4. Etat général du lecteur (1 chiffre) :

0 : Bon état général

1 : Lecteur souillé

2 : Lecteur abîmé

3 : Lecteur hors d'état

(suite)

| | |
|---|---|
| | 4 : Lecteur abîmé - A échanger |
| | 9 : Appareil inaccessible |
| 5. Contrôle hebdomadaire effectué par le personnel soignant (1 chiffre) : | |
| | 0 : Régulier |
| | 1 : Irrégulier |
| | 3 : Feuille non disponible |
| | 4 : Jamais fait |
| | 9 : Non contrôlé |
| 6. Solutions de contrôle (1 chiffre) : | 0 : Correctes |
| | 1 : Date de péremption du mois en cours |
| | 2 : Solutions périmées |
| | 3 : Solutions absentes |
| | 4 : Date d'ouverture non spécifiée |
| | 5 : Utilisation > 3 mois (à changer) |
| | 9 : Non contrôlées |
| 7. Présence de la procédure (1 chiffre) : | 0 : notice d'utilisation disponible |
| | 1 : notice d'utilisation non disponible |
| | 2 : notice absente |
| | 9 : non contrôlée |
| 8. N° lot des bandelettes (6 chiffres : numéro de lot inscrit au dos des bandelettes) | |
| 9. Etat et stockage des bandelettes (1 chiffre) : | 0 : Bon état |
| | 1 : Absence de bandelettes |
| | 2 : Plus d'un lot en cours |
| | 3 : Bandelettes non autorisées |
| | 9 : Non contrôlé |
| 10. Date de péremption des bandelettes (1 chiffre) : | |
| 0 : Date non dépassée | |
| | 1 : Date du mois en cours |
| | 2 : Date dépassée |
| | 3 : Absence de bandelettes |
| | 9 : Non contrôlée |
| 11. Electrode de calibration (1 chiffre) : | 0 : Présente |
| | 1 : Absente |
| | 2 : Incompatible |
| | 3 : A renouveler |
| | 9 : Non contrôlée |
| 12. Calibrage du lecteur (1 chiffre indiquant si le N° de lot des bandelettes correspond au code de calibrage du lecteur) : | 0 : Correcte |
| | 1 : Incorrecte |
| | 2 : Erronée |
| | 3 : A refaire |
| | 9 : Non contrôlé |

Avantageusement, la saisie de la valeur mesurée par l'appareil d'analyse délocalisé est aussi codée dans cette séquence de saisie sous la forme d'une treizième rubrique codée :

13. Résultat contrôle lecteur (X chiffres, par exemple X=3) : Noter le résultat QTD[i] du contrôle affiché sur le lecteur i sous forme de X chiffres (ex. : 045 pour 04,5 mmol/l).

En utilisant ces données qualitatives et quantitatives associées, l'invention permet une évaluation des performances des appareils d'analyse délocalisée en tenant compte du contexte d'utilisation.

Selon l'invention, les données qualitatives sont expertisées à l'aide d'un questionnaire tel que celui présenté ci-dessus. Ce questionnaire peut être créé et modulable par l'utilisateur lui-même. Chaque réponse est également modulable. En particulier, chaque élément enregistré peut ou non être affecté d'un score permettant l'expertise automatique de chaque réponse. Les données issues du contrôle sont de nature différente, qualitative ou quantitative, mais il peut, dans les deux cas, leur être affecté un score.

Le tableau ci-dessous donne un exemple de questionnaire avec deux questions comprenant pour chacune un ensemble de réponses caractérisées par un score. L'attribution de scores, concernant ici le contrôle d'un lecteur de glycémie capillaire Medisense OPTIUM, est faite pour chaque réponse du questionnaire à choix multiple.

Chaque questionnaire rempli comporte un choix de réponses et donc un score cumulé permettant de caractériser l'ensemble de l'observation lorsque les scores sont judicieusement choisis. Ici, un maximum de 0 indique d'emblée une bonne qualité et un score différent de 0 renseigne sur l'importance des problèmes.

Avantageusement, chaque score de chaque réponse pourra servir de loi ou de classement ou de recherche en mode historique ou archive.

Chaque score est inclus dans un score global permettant un système de validation rapide.

| Questions : | Ordre | Question | Score | Type |
|---|---|---|---|---|
| | 1 | Température | | Choix multiples |
| | | 18°C - 20°C | 0 | |
| | | 20°C - 22°C | 0 | |
| | | 22°C - 24°C | 0 | |
| | | 24°C - 26°C | 1 | |
| | | 26°C - 28°C | 2 | |
| | | 28°C - 30°C | 3 | |
| | | Supérieur à 30°C : ALERTE | 4 | |
| | | Inférieur à 18°C : ALERTE | 5 | |
| | 2 | Hygrométrie | | Choix multiples |
| | | 10 % - 15 % : ALERTE | 2 | |
| | | 15 % - 20 % : ALERTE | 1 | |
| | | 20 % - 30 % : | 0 | |
| | | 30 % - 50 % : | 0 | |
| | | 50 % - 60 % : | 0 | |
| | | 60 % - 70 % : ALERTE | 3 | |
| | | Supérieur à 80 % : ALERTE | 4 | |
| | | Inférieur à 10 % : ALERTE | 5 | |

Pour chaque question du questionnaire à choix multiple, l'utilisateur se voit présenter plusieurs fenêtres ou cases à cocher. Lorsque la réponse à la question peut être soit choisie parmi un certain nombre de possibilités (choix multiple) ou dans de manière libre, les deux possibilités sont avantageusement offertes à l'utilisateur sous la forme d'une case à cocher : soit « choix multiple », soit « champ libre ».

L'utilisateur choisit alors un des items du choix multiple ou saisit une valeur libre dans une fenêtre affichée à cet effet sur l'écran de l'appareil de contrôle ou du terminal qui est associé à l'appareil de contrôle.

Le score qui correspond à cette saisie ou à ce choix d'un item s'affiche alors avantageusement dans une fenêtre voisine. Il peut aussi s'afficher uniquement le score global tenant compte de la saisie ou du choix de l'item.

Dans l'exemple donné ci-dessus pour la température, de 18°C à 24°C, l'appareil est dans des conditions normales de fonctionnement et le score est alors nul. Ce score augmente au fur et à mesure que la température observée augmente pour arriver aux scores 4 et 5 qui déclenchent une alerte.

Concernant l'hygrométrie, entre 20 et 60%, le fonctionnement de l'appareil de contrôle est attendu normal. En dehors de ces conditions, le fonctionnement correct est menacé et des alertes sont déclenchées dès qu'un score égal à 2 est atteint.

Une fenêtre de paramétrage est avantageusement proposée à l'utilisateur pour qu'à chaque question, il associe, à chaque fourchette de valeur, un score et à un score donné, une alerte éventuelle.

Une telle fenêtre de paramétrage permet que chaque utilisateur puisse choisir dans quelles limites les conditions environnementales sont acceptables.

Le score associé à chaque question peut entrainer une alerte et par conséquent mener à la révision ou au retrait d'un appareil.

En outre, un score global est avantageusement calculé. Il est cumulatif et peut être, par exemple la somme des scores individuels des questions.

Un score nul signalera que l'appareil est dans les conditions idéales de fonctionnement. Une validation rapide de l'appareil est alors réalisée.

Un score non nul mais faible, par exemple égal à 2, à cause d'une température observée trop élevée, signale des conditions acceptables de fonctionnement. Il est alors possible de valider rapidement l'utilisation de l'appareil.

Lorsqu'un score plus élevé est observé, par exemple 7, il existe un problème à approfondir. Une validation avec contrôles approfondis est alors conseillée.

Enfin, lorsqu'un score par exemple supérieur à 10, par exemple 12, est atteint, il est nécessaire de faire une validation approfondie de l'appareil.

Le questionnaire comprend avantageusement une question au sujet de l'entretien de l'appareil. Dans ce cas, le signalement d'un appareil souillé, à l'aide d'une fenêtre dédiée, sera associé à un score de 10 afin de déclencher forcément l'alerte.

Avec l'invention, il est possible d'offrir à l'utilisateur un tableau comprenant une liste d'appareils de biologie délocalisée à contrôler avec un score associé à chacun de ces appareils.

L'opérateur du contrôle des appareils de biologie délocalisée n'a alors qu'à regarder la colonne des scores et à repérer le ou les appareil(s) qui posent problème.

Les scores peuvent aussi être présentés sous la forme d'un ensemble de points disséminés le long d'un axe des abscisses, leurs scores associés étant portés sur l'axe des ordonnées. On verra qu'une telle présentation est particulièrement adaptée pour vérifier l'exactitude des mesures effectuées par l'appareil.

Aussi, un score global pour chaque expertise permet une validation rapide, automatique et globale d'un ensemble de données.

Comme explicité ci-dessus, au sujet des données qualitatives, chaque biologiste peut construire des questionnaires destinés à recueillir des données numériques ou non relatives à l'environnement et à la sécurisation de l'utilisation des matériels de biologie délocalisée.

Chaque question comporte un choix de réponses qui sont chacune affectée d'un score qui, lui-même, peut être associé ou non à une ou plusieurs couleurs, choisis à la convenance de chaque biologiste et selon le caractère intuitif donné à l'information. Par exemple, chaque réponse peut être affecté d'un score et d'une couleur traduisant le degré d'importance de la réponse : vert = ok, orange = attention, rouge = alerte. Dès lors qu'une question sera associée à la couleur rouge, cela signifiera directement pour le biologiste que cet appareil ne doit plus être utilisé et il mettra alors en oeuvre les actions nécessaires.

Chaque questionnaire et, donc, chaque suite de question présente un score global affecté d'une ou plusieurs couleurs reflétant les différentes réponses sélectionnées au moment de l'expertise.

Chaque questionnaire rempli sur le lieu du contrôle enregistre les différentes réponses et ces données sont transmises par connexion informatique dans la base de données. Cette base de données intègre les résultats et peut les exprimer notamment sous la forme d'un score détaillé ou global.

Le score global avec une ou plusieurs couleurs permet de présenter les données issues du contrôle de manière visuelle, intuitive et rapide permettant au biologiste ou au technicien de décider quasi instantanément si l'expertise demande une validation rapide, incluant par exemple une impression et/ou un envoi immédiat du compte rendu, ou une validation approfondie, incluant une vérification de la saisie des données, de leur interprétation et des conclusions en préalable à l'impression du compte rendu.

Le score détaillé avec ou sans couleur permet une analyse statistique des différentes réponses d'un questionnaire donné et permet aussi une analyse rétrospective performante et détaillée des données qualitatives accumulées.

Au sujet des données quantitatives, les résultats obtenus sont comparés à des fourchettes de valeurs qui, par exemple, permettent de décider entre les items suivants : acceptation des valeurs obtenues, nouveau contrôle du matériel ou non-acceptation /exclusion du matériel. Les résultats obtenus peuvent aussi être comparés à des valeurs cibles à la suite, par exemple du calcul d'un écart ou du calcul d'un indice.

Ces comparaisons entraînent une conclusion pouvant être affectée d'un score associé ou non à une ou plusieurs couleurs : conclusion 1: score 1, conclusion 2 : score 2, conclusion n : score n.... Ce score, éventuellement avec couleurs, peut être traité de façon identique à celui des données qualitatives.

Le score est alors avantageusement nul lorsque la mesure respecte le biais qui est associé à l'appareil de biologie délocalisée. Il est plus ou moins élevé en fonction de la valeur de l'écart observé pour un appareil donné.

Ces scores sont avantageusement présentés sous la forme d'un ensemble de points disséminés le long d'un axe des abscisses, leurs scores associés étant portés sur l'axe des ordonnées. Sur ce graphe, la zone d'écart acceptable pourra être grise, la zone d'écart non acceptable rose et une zone intermédiaire jaune.

Lorsque le score, représentatif de l'écart, sort de la fourchette de valeur préalablement définie, on voit alors d'un coup d'oeil quels sont les appareils dont l'exactitude est en dehors d'une fourchette d'écart acceptable. En affichant un identifiant de l'appareil concerné à proximité de chaque point, la validation de l'exactitude de l'appareil est alors très rapide.

On dispose alors d'un système global de traitement des données qualitatives et quantitatives des données issues de l'assurance qualité des analyses de biologie délocalisée indépendamment des systèmes graphiques couramment utilisés en statistiques.

On sait aussi que les alertes sanitaires émises par les autorités référentes, dont l'AFSSAPS en France, ont pour but de rechercher la présence éventuelle sur le lieu d'utilisation, de matériels et consommables en fonction de leur marque, modèle, n.° de série, n° de lot, référence, date de péremption, date de fabrication ou tout autre mode d'identification, dont des codes à barres ou autres, souvent dans le but de procéder à leur retrait et donc à l'arrêt de leur utilisation.

L'invention permet le suivi des matériels utilisés grâce au codage de données qualitatives relatives au fonctionnement et à l'utilisation de l'appareil. Dans une de ses applications, l'invention permet donc de répercuter ces alertes rapidement sur le terrain. En outre, cette répercussion peut être automatisée en paramétrant le calcul du score de telle manière que l'utilisation d'un matériel donné faisant l'objet d'une alerte sanitaire, par exemple l'utilisation d'un lot de consommables défectueux pour l'utilisation d'un appareil de biologie délocalisée donné, donne un score signifiant que l'appareil ne doit plus être utilisé.

Cette application de l'invention permet une grande sécurisation des soins du patient, notamment par les retraits de matériel par les laboratoires fournisseurs en raison d'une anomalie.

Il s'agit d'une information descendante à partir de la traçabilité offerte par l'invention.

Un des caractères innovants majeurs de l'invention est de permettre une validation accélérée d'un ou de plusieurs appareils contrôlés et le traitement statistique de données qualitatives. En outre, l'invention permet une expertise nomade sur le lieu du contrôle et donc la sécurisation des conditions d'utilisation du matériel.

En particulier, l'invention permet la mise en oeuvre de validations rapides des données de l'assurance qualité des appareils de biologie délocalisée en autorisant l'analyse de données qualitatives et quantitatives issues des expertises sur le lieu même de l'utilisation de l'appareil d'analyse biologique délocalisée.

Dans son application, l'invention permet de satisfaire aux exigences de la norme ISO 22870 : 2006 « Analyses De Biologie Délocalisée (ADBD) - Exigences concernant la qualité et la compétence » spécifique aux appareils de biologie délocalisée qui sont des appareils particuliers pour lesquels se posent des problèmes distincts de ceux rencontrés pour les appareils de laboratoire.

Avantageusement, l'appareil de contrôle 3 comprend des moyens pour gérer un ou des abonnements au service de base de données et de traitement des données offerts par le système informatique centralisateur 4. La gestion d'un tel abonnement utilise les moyens connus de l'homme du métier pour gérer un abonnement à un service réseau, par exemple sur le réseau internet.

Les moyens apportés par l'invention permettent de comparer les résultats de mesure obtenus dans des contextes particuliers à l'aide d'une base de données à partir de laquelle on peut élargir les données de l'analyse ou au contraire sélectionner des données d'analyse particulières. Ces comparaisons permettent de conclure quant à l'état des appareils d'analyse délocalisée, de commenter et documenter ces conclusions. Ces conclusions sont rapides et permettent donc des diagnostics précoces des appareils d'analyse délocalisée.

On remarque enfin que diverses mises en oeuvre peuvent être réalisées selon les principes de l'invention. Notamment, les moyens de détection peuvent être matériellement intégrés au capteur, comme décrit dans la figure 1, ou en liaison avec celui-ci, comme décrit dans la figure 4.

## Revendications

**1.** Procédé de vérification de l'état et des performances d'appareils d'analyse biologique délocalisée [i=10 à 14] destiné à être mis en oeuvre *in situ* à partir d'un appareil dit de contrôle [2] portable , à savoir sur le lieu du dit appareil d'analyse biologique délocalisée dans un lieu distinct d'un laboratoire d'analyse médicale, comprenant les étapes de :

a) saisie ou réception [E1] *in situ* de données qualitatives [QLD[i]] caractérisant un appareil d'analyse biologique délocalisée [i] devant être contrôlé et caractérisant l'environnement dans lequel se trouve cet appareil, les dites données qualitatives comprenant l'identification et la localisation de l'appareil biologique délocalisée ainsi que des données choisies au moins parmi des données relatives à l'état, l'entretien, la fréquence d'utilisation et la méthode d'emploi de l'appareil d'analyse biologique délocalisée et l'état de produits et consommables et/ou de solution dite de contrôle pour l'utilisation de l'appareil d'analyse biologique délocalisée,

b) saisie ou réception [E0] in situ de la valeur [QTD[i]] d'une variable biologique d'une dite solution de contrôle [SC] mesurée par l'appareil de biologie délocalisée [i] devant être contrôlé,

c) acquisition [E2] de la valeur [$QTD_{CTL}$] de la variable biologique de la solution de contrôle [SC] mesurée par un appareil d'analyse biologique de référence [3],

d) transmission [E3] de la valeur [QTD[i]] saisie ou reçue, de la valeur [$QTD_{CTL}$] acquise et des données qualitatives [QLD[i]] saisies ou reçues vers un système informatique centralisateur [4],

e) détermination [E4], au sein du système informatique centralisateur [4], d'un biais [B[i]] attendu entre la valeur [QTD[i]] de la variable biologique saisie ou reçue et la valeur [QTD$_{CTL}$] mesurée avec l'appareil de référence [3] en fonction des données qualitatives [QLD[i]] saisies ou reçues, dont celles renseignant l'environnement dans lequel l'appareil est utilisé, et de données de comparaison [DC] stockées dans le système informatique [4],

f) comparaison [E5] de la valeur [QTD[i]] mesurée sur l'appareil de biologie délocalisée avec la valeur [QTD$_{CTL}$] mesurée par l'appareil de référence [3] en tenant compte du biais [B[i]],

g) stockage [E6] de données de comparaison [DC] relatives à la comparaison effectuée dans le système informatique [4] associées à des données qualitatives,

h) élaboration, au sein du système informatique, d'un compte rendu [C-R] de contrôle,

i) envoi [E7] du compte rendu [C-R] vers l'appareil de contrôle [2,3'] ou vers un terminal informatique associé à l'appareil de contrôle [2,3'].

2. Procédé de contrôle d'appareils d'analyse biologique délocalisée [i=10 à 14] selon la revendication 1, **caractérisé en ce que** l'appareil de contrôle [3'] est également un appareil d'analyse biologique de référence sur lequel, lors de l'étape d'acquisition [E2], est directement mesurée la valeur [QTD$_{CTL}$] de la variable biologique de la solution de contrôle [SC].

3. Procédé selon la revendication 1, **caractérisé en ce qu'**il comprend une étape d'alerte déclenchée en fonction du contenu du compte rendu [C-R] reçu en provenance du système informatique [4].

4. Procédé selon l'une des revendications précédentes, **caractérisé en ce que** le stockage des données de comparaison [DC] associées à des données qualitatives est réalisé sous la forme d'une base de données.

5. Procédé selon l'une des revendications précédentes, **caractérisé en ce que** le système informatique [4] est constitué d'une seule machine ou d'une pluralité reliée entre elles par un réseau.

6. Procédé selon l'une des revendications précédentes, **caractérisé en ce qu'**il est apte à fonctionner en mode initialisation dont le but est de définir un biais [B[i]] pour chaque appareil d'analyse biologique délocalisée [i] en fonction de la solution de contrôle [SC] utilisée, le biais [B[i]] étant défini égal à la différence entre la valeur [QTD[i]] de la variable mesurée avec l'appareil d'analyse biologique délocalisée [i] et la valeur [QTD$_{CTL}$] mesurée avec l'appareil de référence [3,3'] ou à la moyenne de cette différence sur une pluralité de mesures par l'appareil d'analyse délocalisée [i] et le biais [B[i]] étant stocké en tant que données de comparaison [DC].

7. Procédé selon l'une des revendications précédentes, **caractérisé en ce que** l'étape de stockage [E6] de données de comparaison [DC] n'est réalisée qu'en début d'utilisation et de contrôle d'un appareil d'analyse biologique délocalisée [i].

8. Procédé selon l'une des revendications précédentes, **caractérisé en ce que** les données qualitatives [QLD[i]] incluent des données qualifiant la solution de contrôle [SC].

9. Procédé selon l'une des revendications précédentes, **caractérisé en ce que** la variable biologique est choisie parmi les variables suivantes : glycémie, gaz du sang, hématocrite, lactates, ionogramme sanguin ou urinaire, glucose urinaire, créatinine, hémoglobineA1c, acide urique, cholestérol, triglycérides.

10. Procédé selon l'une des revendications précédentes, **caractérisé en ce qu'**un score est associé à chaque donnée qualitative renseignée et **en ce qu'**un score est associé à chaque donnée quantitative en fonction de l'étape de comparaison et **en ce qu'**un score final, somme ou calcul issu des scores des données qualitatives et quantitatives, constitue un des éléments du compte-rendu ou permet de déclencher ou non une étape d'alerte.

11. Procédé selon la revendication 10, **caractérisé en ce qu'**il comprend une étape d'association automatique de couleurs prédéfinies à chaque score, ces couleurs permettant de caractériser instantanément des scores élevés, limites ou anormaux et de traiter spécifiquement chaque score selon son importance relative.

12. Appareil de contrôle [2,3'] portable à partir duquel un procédé selon l'une des revendications 1 à 11 est mis en oeuvre in situ, à savoir sur le lieu du dit appareil d'analyse biologique délocalisée dans un lieu distinct d'un laboratoire d'analyse médicale, comprenant pour cela :

- des moyens de saisie [33] ou de réception de données qualitatives [QLD[i]] caractérisant un appareil de biologie délocalisée [i] devant être contrôlé et caractérisant l'environnement dans lequel se trouve cet appareil, les dites données qualitatives comprenant l'identification et la localisation de l'appareil biologique délocalisée ainsi que des données choisies au moins parmi des données relatives à l'état, l'entretien, la fréquence d'utilisation et la méthode d'emploi de l'appareil d'analyse biologique délocalisée et l'état de produits et consommables et/ou de solution dite de contrôle pour l'utilisation de l'appareil d'analyse biologique délocalisée,

- des moyens de saisie [33] ou de réception de la valeur [QTD[i]] d'une variable biologique d'une dite solution de contrôle [SC] mesurée par l'appareil de biologie délocalisée [i] devant être contrôlé,

- des moyens d'acquisition de la valeur [$QTD_{CTL}$] de la variable biologique de la solution de contrôle [SC] mesurée par un appareil d'analyse biologique de référence [3]

- des moyens de transmission [37] de la valeur [QTD[i]] saisie ou reçue, de la valeur [$QTD_{CTL}$] mesurée et des données qualitatives [QLD[i]] saisies ou reçues vers un système informatique centralisateur [4] apte à déterminer un biais [B[i]] attendu entre la valeur [QTD[i]] de la variable biologique saisie ou reçue et la valeur [$QTD_{CTL}$] mesurée avec l'appareil de référence [3] en fonction des données qualitatives [QLD[i]] saisies ou reçues, dont celles renseignant l'environnement dans lequel l'appareil est utilisé, et de données de comparaison [DC] stockées dans le système informatique [4], à comparer la valeur [QTD[i]] mesurée sur l'appareil de biologie délocalisée avec la valeur [$QTD_{CTL}$] mesurée par l'appareil de référence [3] en tenant compte du biais [B[i]], à stocker des données de comparaison [DC] relatives à la comparaison effectuée associées à des données qualitatives [QLD[i]], à élaborer un compte rendu [C-R] de contrôle et à l'envoyer vers l'appareil de contrôle [2,3'] ou un terminal informatique associé à cet appareil de contrôle [2,3'].

13. Appareil de contrôle [3'] selon la revendication 11, **caractérisé en ce qu'**il comprend des moyens de mesure [34] de la valeur [$QTD_{CTL}$] de la variable biologique de la solution de contrôle [SC], et **en ce qu'**il est également l'appareil d'analyse biologique de référence [3'].

14. Appareil de contrôle selon la revendication 11, **caractérisé en ce qu'**il comprend en outre des moyens de réception [37] du compte rendu [C-R] de contrôle.

15. Appareil de contrôle selon la revendication 13, **caractérisé en ce qu'**il comprend en outre des moyens d'alerte déclenchés en fonction du contenu du compte rendu [C-R].

16. Système informatique centralisateur [4] apte à communiquer avec une pluralité d'appareils de contrôle [2,3'] selon l'une des revendications 12 à 15, le système informatique [4] comprenant :

- des moyens [43] pour recevoir la valeur [QTD[i]] saisie ou reçue par l'appareil de référence [3,3'], la valeur [$QTD_{CTL}$] mesurée avec un appareil de référence [3] et les données qualitatives [QLD[i]], transmises par l'appareil de contrôle [2,3'],

- des moyens de détermination [44] d'un biais [B[i]] attendu entre la valeur [QTD[i]] de la variable biologique saisie ou reçue et la valeur [$QTD_{CTL}$] mesurée avec l'appareil de référence [3,3'] en fonction des données qualitatives [QLD[i]] saisies et de données de comparaison [DC] stockées dans le système informatique [4],

- des moyens de comparaison [45] de la valeur [QTD[i]] mesurée sur l'appareil de biologie délocalisée [i] avec la valeur [$QTD_{CTL}$] mesurée par l'appareil de référence [3] en tenant compte du biais [B[i]],

- des moyens de stockage [42] pour stocker des données de comparaison [DC] relatives à la comparaison effectuée,

- des moyens pour élaborer un compte rendu [C-R] de contrôle,

- des moyens de transmission [43] pour envoyer le compte rendu [C-R] de contrôle vers l'appareil de contrôle [2,3'] ou vers un terminal informatique associé à cet appareil de contrôle [2,3'].

**Patentansprüche**

1. Verfahren zur Überprüfung des Zustands und der Leistungen von Geräten für eine delokalisierte biologische Analyse [i = 10 bis 14], das dazu bestimmt ist, *in situ* von einem tragbaren Kontrollgerät [2] umgesetzt zu werden, das heißt am Ort des Geräts für die delokalisierte biologische Analyse an einem Ort, der von einem medizinischen Analyselabor verschieden ist, umfassend die Schritte des:

a) Erfassens oder Empfangens [E1] von qualitativen Daten [QLD[i]] *in situ,* die ein Gerät für eine delokalisierte biologische Analyse [i] charakterisieren, das kontrolliert werden muss, und die die Umgebung charakterisieren, in der sich dieses Gerät befindet, wobei die qualitativen Daten die Identifizierung und Lokalisierung des Geräts für die delokalisierte biologische Analyse und Daten aufweisen, die mindestens aus Daten ausgewählt sind, die sich auf den Zustand, die Wartung, die Häufigkeit der Verwendung und die Methode der Verwendung des Geräts für die delokalisierte biologische Analyse und den Zustand von Produkten und Verbrauchsmaterialien und/oder von einer Kontrolllösung für die Verwendung des Geräts für die delokalisierte biologische Analyse beziehen,

b) Erfassens oder Empfangens [E0] *in situ* des Wertes [QTD[i]] einer biologischen Variablen einer Kontrolllösung [SC], die von dem Gerät für die delokalisierte biologische Analyse [i], das kontrolliert werden muss, gemessen wird,

c) Erwerbens [E2] des Wertes [$QTD_{CTL}$] der biologischen Variablen der Kontrolllösung [SC], die von einem Referenzgerät für eine biologische Analyse [3] gemessen wird,

d) Übertragens [E3] des erfassten und empfangenen Wertes [QTD[i]], des erworbenen Wertes [$QTD_{CTL}$] und der erfassten oder empfangenen qualitativen Daten [QLD[i]] an ein zentralistisches Informatiksystem [4],

e) Bestimmens [E4] in dem zentralistischen Informatiksystem [4] eines erwarteten Biais [B[i]] zwischen dem Wert [QTD[i]] der erfassten und empfangenen biologischen Variablen und dem Wert [$QTD_{CTL}$], der mit dem Referenzgerät [3] gemessen wird, in Abhängigkeit von den erfassten oder empfangenen qualitativen Daten [QLD[i]], darunter jene, die über die Umgebung informieren, in der das Gerät verwendet wird, und von Vergleichsdaten [DC], die in dem Informatiksystem [4] gespeichert werden,

f) Vergleichens [E5] des Wertes [QTD[i]], der auf dem Gerät für die delokalisierte biologische Analyse gemessen wird, mit dem Wert [$QTD_{CTL}$], der von dem Referenzgerät [3] gemessen wird, unter Berücksichtigung des Biais [B[i]],

g) Speicherns [E6] von Vergleichsdaten [DC] in Bezug auf den durchgeführten Vergleich in dem Informatiksystem [4], die mit qualitativen Daten verbunden werden,

h) Erarbeitens in dem Informatiksystem eines Kontrollberichts [C-R],

i) Sendens [E7] des Berichts [C-R] an das Kontrollgerät [2, 3'] oder an ein Computerendgerät, das mit dem Kontrollgerät [2, 3'] verbunden wird.

2. Verfahren zur Kontrolle von Geräten für eine delokalisierte biologische Analyse [i = 10 bis 14] gemäß Anspruch 1, **dadurch gekennzeichnet, dass** das Kontrollgerät [3'] auch ein Referenzgerät für eine biologische Analyse ist, auf dem bei dem Schritt des Erwerbens [E2] der Wert [$QTD_{CTL}$] der biologischen Variablen der Kontrolllösung [SC] direkt gemessen wird.

3. Verfahren gemäß Anspruch 1, **dadurch gekennzeichnet, dass** es einen Alarmschritt aufweist, der in Abhängigkeit von dem Inhalt des Berichts [C-R], der von dem Informatiksystem [4] empfangen wird, ausgelöst wird.

4. Verfahren gemäß einem der vorhergehenden Ansprüche, **dadurch gekennzeichnet, dass** das Speichern von Vergleichsdaten [DC], die mit qualitativen Daten verbunden werden, in Form von einer Datenbank durchgeführt wird.

5. Verfahren gemäß einem der vorhergehenden Ansprüche, **dadurch gekennzeichnet, dass** das Informatiksystem [4] aus einer einzigen Maschine oder aus mehreren Maschinen gebildet wird, die durch ein Netz miteinander verbunden werden.

6. Verfahren gemäß einem der vorhergehenden Ansprüche, **dadurch gekennzeichnet**, das es geeignet ist, im Initialisierungsmodus zu funktionieren, dessen Ziel es ist, ein Biais [B[i]] für jedes Gerät für eine delokalisierte biologische Analyse [i] in Abhängigkeit von der verwendeten Kontrolllösung [SC] zu definieren, wobei der Biais [B[i]] gleich der Differenz zwischen dem Wert [QTD[i]] der Variablen, die mit dem Gerät für die delokalisierte biologische Analyse [i] gemessen wird, und dem Wert [$QTD_{CTL}$], der mit dem Referenzgerät [3, 3'] gemessen wird, oder gleich dem Mittelwert dieser Differenz für mehrere Messungen durch das Gerät für die delokalisierte Analyse [i] definiert wird, und wobei der Biais [B[i]] als Vergleichsdaten [DC] gespeichert wird.

7. Verfahren gemäß einem der vorhergehenden Ansprüche, **dadurch gekennzeichnet, dass** der Schritt des Speicherns [E6] von Vergleichsdaten [DC] nur zu Beginn der Verwendung und der Kontrolle eines Geräts für die delokalisierte biologische Analyse [i] durchgeführt wird.

8. Verfahren gemäß einem der vorhergehenden Ansprüche, **dadurch gekennzeichnet, dass** die qualitativen Daten [QLD[i]] Daten umfassen, die die Kontrolllösung [SC] qualifizieren.

9. Verfahren gemäß einem der vorhergehenden Ansprüche, **dadurch gekennzeichnet, dass** die biologische Variable aus den folgenden Variablen ausgewählt wird: Blutzucker, Blutgas, Hämatokrit, Lactate, Blut- oder Urinionogramm, Uringlukose, Kreatinin, Hämoglobin A1c, Harnsäure, Cholesterin, Triglyceride.

**10.** Verfahren gemäß einem der vorhergehenden Ansprüche, **dadurch gekennzeichnet, dass** jedem angezeigten qualitativen Datum eine Punktzahl zugeordnet wird und dass jedem quantitativen Datum in Abhängigkeit von dem Schritt des Vergleiches eine Punktzahl zugeordnet wird und dass eine endgültige Punktzahl, Summe oder Berechnung, die aus den Punktzahlen der qualitativen und quantitativen Daten hervorgegangen ist, eines der Elemente des Berichts bildet oder ermöglicht, einen Alarmschritt auszulösen oder nicht.

**11.** Verfahren gemäß Anspruch 10, **dadurch gekennzeichnet, dass** es einen Schritt des automatischen Zuordnens von vorbestimmten Farben zu jeder Punktzahl aufweist, wobei diese Farben ermöglichen, hohe, extreme oder anomale Punktzahlen sofort zu charakterisieren und jede Punktzahl spezifisch gemäß ihrer relativen Bedeutung zu bearbeiten.

**12.** Tragbares Kontrollgerät [2, 3'], von dem aus ein Verfahren gemäß einem der Ansprüche 1 bis 11 *in situ* durchgeführt wird, das heißt am Ort des Geräts für die delokalisierte biologische Analyse an einem Ort, der von einem medizinischen Analyselabor verschieden ist, zu diesem Zweck umfassend:

- Mittel zum Erfassen [33] oder Empfangen von qualitativen Daten [QLD[i]], die ein Gerät für eine delokalisierte biologische Analyse [i] charakterisieren, das kontrolliert werden muss, und die die Umgebung charakterisieren, in der sich dieses Gerät befindet, wobei die qualitativen Daten die Identifizierung und Lokalisierung des Geräts für die delokalisierte biologische Analyse und Daten aufweisen, die mindestens aus Daten ausgewählt sind, die sich auf den Zustand, die Wartung, die Häufigkeit der Verwendung und die Methode der Verwendung des Geräts für die delokalisierte biologische Analyse und den Zustand von Produkten und Verbrauchsmaterialien und/oder von einer Kontrolllösung für die Verwendung des Geräts für die delokalisierte biologische Analyse beziehen,
- Mitte! zum Erfassen [33] oder Empfangen des Wertes [QTD[i]] einer biologischen Variablen einer Kontrolllösung [SC], die von dem Gerät für die delokalisierte biologische Analyse [i], das kontrolliert werden muss, gemessen wird,
- Mittel zum Erwerben des Wertes [$QTD_{CTL}$] der biologischen Variablen der Kontrolllösung [SC], die von einem Referenzgerät für eine biologische Analyse [3] gemessen wird,
- Mittel zum Übertragen [37] des erfassten oder empfangenen Wertes [QTD[i]], des gemessenen Wertes [$QTD_{CTL}$] und der erfassten oder empfangenen qualitativen Daten [QLD[i]] an ein zentralistisches Informatiksystem [4], das geeignet ist, einen erwarteten Biais [B[i]] zwischen dem Wert [QTD[i]] der erfassten und empfangenen biologischen Variablen und dem Wert [$QTD_{CTL}$], der mit dem Referenzgerät [3] gemessen wird, in Abhängigkeit von den erfassten oder empfangenen qualitativen Daten [QLD[i]], darunter jene, die über die Umgebung informieren, in der das Gerät verwendet wird, und von Vergleichsdaten [DC], die in dem Informatiksystem [4] gespeichert sind, zu bestimmen, den Wert [QTD[i]], der auf dem Gerät für die delokalisierte biologische Analyse gemessen wird, mit dem Wert [$QTD_{CTL}$], der von dem Referenzgerät [3] gemessen wird, unter Berücksichtigung des Biais [B[i]] zu vergleichen, Vergleichsdaten [DC] in Bezug auf den durchgeführten Vergleich, die qualitativen Daten [QLD[i]] zugeordnet sind, zu speichern, einen Kontrollbericht [C-R] zu erstellen und ihn an das Kontrollgerät [2, 3'] oder an ein Computerendgerät, das diesem Kontrollgerät [2, 3'] zugeordnet ist, zu senden.

**13.** Kontrollgerät [3'] gemäß Anspruch 11, **dadurch gekennzeichnet, dass** es Mittel zum Messen [34] des Wertes [$QTD_{CTL}$] der biologischen Variablen der Kontrolllösung [SC] aufweist und dass es ebenfalls das Referenzgerät für die biologische Analyse [3'] ist.

**14.** Kontrollgerät gemäß Anspruch 11, **dadurch gekennzeichnet, dass** es ferner Mittel zum Empfangen [37] des Kontrollberichts [C-R] aufweist.

**15.** Kontrollgerät gemäß Anspruch 13, **dadurch gekennzeichnet, dass** es ferner Alarmmittel aufweist, die in Abhängigkeit von dem Inhalt des Berichts [C-R] ausgelöst werden.

**16.** Zentralistisches Informatiksystem [4], das geeignet ist, mit mehreren Kontrollgeräten [2, 3'] gemäß einem der Ansprüche 12 bis 15 zu kommunizieren, wobei das Informatiksystem [4] aufweist:

- Mittel [43] zum Empfangen des Wertes [QTD[i]], der von dem Referenzgerät [3, 31] erfasst und empfangen wird, des Wertes [$QTD_{CTL}$], der mit einem Referenzgerät [3] gemessen wird, und der qualitativen Daten [QLD[i]], die von dem Kontrollgerät [2, 3'] übertragen werden,
- Mittel zum Bestimmen [44] eines erwarteten Biais [B[i]] zwischen dem Wert [QTD[i]] der erfassten und empfangenen biologischen Variablen und dem Wert [QTD$_{CTL}$], der mit dem Referenzgerät [3, 31] gemessen wird, in Abhängigkeit von den erfassten qualitativen Daten [QLD[i]] und von Vergleichsdaten [DC], die in dem Informatiksystem [4] gespeichert sind,

- Mittel zum Vergleichen [45] des Wertes [QTD[i]], der auf dem Gerät für die delokalisierte biologische Analyse [i] gemessen wird, mit dem Wert [QTD$_{CTL}$], der von dem Referenzgerät [3] gemessen wird, unter Berücksichtigung des Biais [B[i]],

- Speichermittel [42], um Vergleichsdaten [DC] in Bezug auf den durchgeführten Vergleich zu speichern,

- Mittel zum Erarbeiten eines Kontrollberichts [C-R],

- Übertragungsmittel [43], um den Kontrollbericht [C-R] an das Kontrollgerät [2, 3'] oder an ein Computerendgerät, das mit diesem Kontrollgerät [2, 3'] verbunden ist, zu senden.

**Claims**

1. A method of inspecting the state and the performance of point-of-care biological test appliances (i = 10 to 14) for on-site implementation using a portable inspection appliance (2), namely , on the spot of said point-of-care biological test appliances, away from a medical analysis laboratory, the method comprising the steps of:

a) on-site inputting or reception (E1) of qualitative data (QLD[i]) characterizing a point-of-care biological test appliance (i) for inspection and characterizing the environment in which the appliance is to be found, said qualification data comprising identification and location of said point-of-care biological appliance as well as data selected from at least data relative to the state, maintenance, frequency, of use, and method of use the point-of-care appliances, and the state of products and consumables and/or of so-called control solution for the use of said point-of-care biological test appliance;

b) on-site inputting or reception (E0) of the value (QTD[i]) of a biological variable of said "control" solution (SC) as measured by the point-of-care biological test appliance (i) for inspection;

c) acquiring (E2) the value (QTD[i]) of the biological variable of the control solution (SC) as measured by a reference biological test appliance (3);

d) transmitting (E3) the input or received value (QTD[i]), the acquired value (QTD$_{CTL}$), and the input or received qualitative data (QLD[i]) to a centralizing computer system (4);

e) within the centralizing computer system (4), determining (E4) a bias (B[i]) that is to be expected between the input or received value (QTD[i]) of the biological variable and the value (QTD$_{CTL}$) as measured by the reference appliance (3), the expected bias being determined as a function of the input or received qualitative data (QLD[i]), including data relating to the environment in which the appliance is used, and of comparison data (DC) stored in the computer system (4);

f) comparing (E5) the value (QTD[i]) as measured by the point-of-care biological test appliance with the value (QTD$_{CTL}$) as measured by the reference appliance (3) while taking the bias (B[i]) into account;

g) storing (E6) comparison data (DC) relating to the comparison performed in the computer system (4), said comparison data being stored in association with the qualitative data;

h) within the computer system, generating a inspection report (C-R); and

i) sending (E7) the report (C-R) to the inspection appliance (2, 3') or to a computer terminal associated with the inspection appliance (2, 3').

2. A method of inspecting point-of-care biological test appliances (i = 10 to 14) according to claim 1, **characterized in that** the inspection appliance (3') is also a reference biological test appliance on which the value (QTD$_{CTL}$) of the biological variable of the control solution (SC) is measured directly during the acquisition step (E2).

3. A method according to claim 1, **characterized in that** it includes a step of triggering a warning as a function of the content of the report (C-R) received from the computer system (4).

4. A method according to any preceding claim, **characterized in that** the comparison data (DC) associated with the qualitative data is stored in the form of a database.

5. A method according to any preceding claim, **characterized in that** the computer system (4) is constituted by a single machine or by a plurality of machines connected together by a network.

6. A method according to any preceding claim, **characterized in that** it is suitable for operating in an initialization mode for the purpose of defining a bias (B[i]) for each point-of-care biological test appliance (i) as a function of the control solution (SC) used, the bias (B[i]) being defined as being equal to the difference between the value (QTD[i]) of the variable as measured by the point-of-care biological test appliance (i) and the value (QTD$_{CTL}$) as measured by the reference appliance (3, 3'), or to the mean of said difference over a plurality of measurements performed by the point-of-care biological test appliance (i), with the bias (B[i]) being stored as part of the comparison data (DC).

7. A method according to any preceding claim, **characterized in that** the step (E6) of storing comparison data (DC) is performed only at the beginning of using and inspecting a point-of-care biological test appliance (i).

8. A method according to any preceding claim, **characterized in that** the qualitative data (QLD[i]) include data qualifying the control solution (SC).

9. A method according to any preceding claim, **characterized in that** the biological variable is selected from the following variables: glycemia, blood gas, hematocrit, lactates, urinary or blood ionogram, urinary glucose, creatinine, hemoglobin A1c, uric acid, cholesterol, triglycerides.

10. A method according to any preceding claim, **characterized in that** a score is associated with each reported qualitative data item, and **in that** a score is associated with each quantitative data item as a function of the comparison step, and **in that** a final score obtained by summing or performing a calculation on the qualitative and quantitative data scores constitutes one of the elements of the report or serves to enable a warning step to be triggered or not triggered.

11. A method according to claim 10, **characterized in that** it includes a step of automatically associating predefined colors with each score, the colors enabling scores that are high, at the limit, or abnormal to be characterized instantly, and enabling each score to be processed specifically depending on its relative importance.

12. A portable inspection appliance (2, 3') from which a method according to any one of claims 1 to 10 is implemented on site, namely, on the spot of said point-of-care biological test appliances, away from a medical analysis laboratory, the appliance comprising:

   • means (33) for inputting or receiving qualitative data (QLD[i]) characterizing a point-of-care biological test appliance (i) for inspection and characterizing the environment in which the appliance is to be found, said qualification data comprising identification and location of said point-of-care biological appliance as well as data selected from at least data relative to the state, maintenance, frequency, of use, and method of use the point-of-care appliances, and the state of products and consumables and/or of so-called control solution for the use of said point-of-care biological test appliance;
   • means (33) for inputting or receiving the value (QTD[i]) of a biological variable of said "control" solution (SC) as measured by the point-of-care biological test appliance (i) for inspection;
   • means for acquiring the value (QTD$_{CTL}$) of the biological variable of the control solution (SC) as measured by a reference biological test appliance (3); and
   • means (37) for transmitting the input or received value (QTD[i]), the measured value (QTD$_{CTL}$), and the input or received qualitative data (QLD[i]) to a centralizing computer system (4) suitable for:

      - determining a bias (B[i]) that is to be expected between the input or received value (QTD[i]) of the biological variable and the value (QTD$_{CTL}$) as measured by the reference appliance (3), the expected bias being determined as a function of the input or received qualitative data (QLD[i]), including data reporting on the environment in which the appliance is used, and of comparison data (DC) stored in the computer system (4);
      - comparing the value (QTD[i]) as measured by the point-of-care biological test appliance with the value (QTD$_{CTL}$) as measured by the reference appliance (3) while taking the bias (B[i]) into account;
      - storing the comparison data (DC) relating to the comparison performed, said comparison data being stored in association with the qualitative data (QLD[i]);
      - generating a inspection report (C-R); and
      - sending the report to the inspection appliance (2, 3') or to a computer terminal associated with said inspection appliance (2, 3').

13. An inspection appliance (3') according to claim 11, **characterized in that** it includes means (34) for measuring the value (QTD$_{CTL}$) of the biological variable of the control solution (SC), and **in that** it also constitutes the reference biological test appliance (3').

14. An inspection appliance according to claim 11, **characterized in that** it further comprises means (37) for receiving the inspection report (C-R).

15. An inspection appliance according to claim 13, **characterized in that** it further comprises warning means triggered as a function of the content of the report (C-R).

16. A centralizing computer system (4) suitable for communicating with a plurality of inspection appliances (2,3') according to any one of claims 11 to 14, the computer system (4) comprising:

> • means (43) for receiving the value (QTD[i]) input to or received by the reference appliance (3), the value (QTD$_{CTL}$) measured by a reference appliance (3), and the qualitative data (QLD[i]) transmitted by the inspection appliance (2, 3');
> • means (44) for determining a bias (B[i]) that is to be expected between the input or received value (QTD[i]) of the biological variable and the value (QTD$_{CTL}$) as measured by the reference appliance (3), the expected bias being determined as a function of the input qualitative data (QLD[i]) and of comparison data (DC) stored in the computer system (4);
> • means (45) for comparing the value (QTD[i]) as measured by the point-of-care biological test appliance (i) with the value (QTD$_{CTL}$) as measured by the reference appliance (3) while taking the bias (B[i]) into account;
> • storage means (42) for storing comparison data (DC) relating to the comparison performed;
> • means for generating a inspection report (C-R); and
> • transmission means (43) for sending the inspection report (C-R) to the inspection appliance (2, 3') or to a computer terminal associated with said inspection appliance (2, 3').

FIG.1a

FIG.1b

FIG.2

FIG.3

FIG.4

FIG.5